(19) Europäisches Patentamt
European Patent Office
Office européen des brevets

(11) Veröffentlichungsnummer: **0 299 913 B1**

(12) **EUROPÄISCHE PATENTSCHRIFT**

(45) Veröffentlichungstag der Patentschrift: **31.03.93**

(51) Int. Cl.5: **C07J 1/00**, A61K 31/565, C07J 41/00

(21) Anmeldenummer: **88730161.2**

(22) Anmeldetag: **15.07.88**

(54) **11Beta-Phenyl-4,9,15-Estratriene, deren Herstellung und diese enthaltende pharmazeutische Präparate.**

(30) Priorität: **16.07.87 DE 3723788**

(43) Veröffentlichungstag der Anmeldung:
**18.01.89 Patentblatt 89/03**

(45) Bekanntmachung des Hinweises auf die Patenterteilung:
**31.03.93 Patentblatt 93/13**

(84) Benannte Vertragsstaaten:
**AT BE CH DE ES FR GB GR IT LI LU NL SE**

(56) Entgegenhaltungen:
**EP-A- 0 051 762**
**EP-A- 0 190 759**
**WO-A-83/03099**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Ottow, Eckhard, Dr.**
**Sonnenallee 124**
**W-1000 Berlin 44(DE)**
Erfinder: **Hofmeister, Helmut, Dr.**
**Weislingenstrasse 4**
**W-1000 Berlin 28(DE)**
Erfinder: **Scholz, Stefan, Dr.**
**Lützenstrasse 9**
**W-1000 Berlin 31(DE)**
Erfinder: **Neef, Günter, Dr.**
**Darmstädter Strasse 9**
**W-1000 Berlin 15(DE)**
Erfinder: **Elger, Walter, Dr.**
**Schorlemer Allee 12B**
**W-1000 Berlin 33(DE)**
Erfinder: **Beier, Sybille, Dr.**
**Uhlandstrasse 121**
**W-1000 Berlin 31(DE)**
Erfinder: **Chwalisz, Krzysztof, Dr.**
**Luzerner Strasse 1d**
**W-1000 Berlin 45(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 299 913 B1

## Beschreibung

Die Erfindung betrifft neue 11ß-Phenyl-4,9,15-estratriene, Verfahren zu deren Herstellung und diese Verbindungen enthaltende pharmazeutische Präparate.

Die erfindungsgemäßen Verbindungen werden durch die allgemeine Formel I charakterisiert

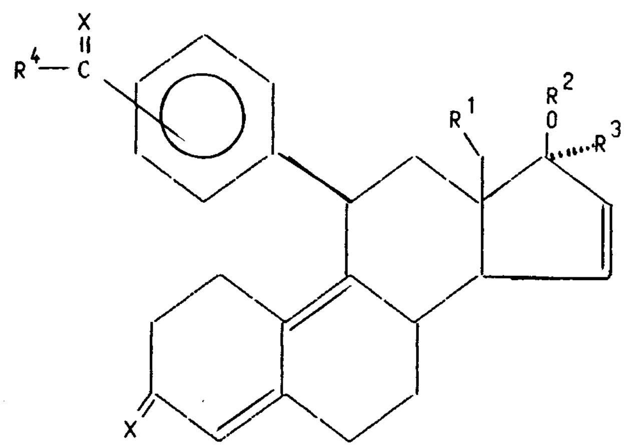

(I),

worin

- X für ein Sauerstoffatom oder eine Hydroxyiminogruppierung N~OH,
- $R^1$ für ein Wasserstoffatom oder eine Methylgruppe,
- $R^2$ für ein Wasserstoffatom, einen Alkylrest oder einen Acylrest mit jeweils 1 bis 10 Kohlenstoffatomen,
- $R^3$ für ein Wasserstoffatom, eine Cyanmethylgruppe, oder den Rest $-(CH_2)_nCH_2Z$, wobei n die Ziffern 0, 1, 2, 3, 4 oder 5 bedeutet und Z = -H oder $-OR^5$ ist mit $R^5$ gleich ein Wasserstoffatom oder ein Alkyl- oder Acylrest mit jeweils 1 bis 10 Kohlenstoffatomen, oder $R^3$ für den Rest $-(CH_2)_mC{\equiv}C-Y$ wobei m = 0,1 oder 2 und Y ein Wasserstoff-, Chlor-, Fluor-, Jod- oder Bromatom, eine Alkyl-, Hydroxyalkyl-, Alkoxyalkyl- oder Acyloxyalkylgruppe mit jeweils 1 bis 10 Kohlenstoffatomen bedeutet,
- $R^4$ für einen geradkettigen oder verzweigten, gesättigten oder ungesättigten Alkylrest mit bis zu 7 Kohlenstoffatomen, oder für einen Phenylrest

stehen.

Die in $R^2$ enthaltenen Alkyl- und Acyl- bzw. die in $R^5$ und Y enthaltenen Alkyl- Acyl- bzw. Alkoxygruppen sollen jeweils 1 bis 10 Kohlenstoffatome haben, wobei die Methyl, Ethyl-, Propyl-, Formyl-, Acetyl-, Propionyl-, Butyryl, Benzoyl-, Methoxy-und Ethoxygruppe bevorzugt sind.

Die in $R^4$ der allgemeinen Formel I enthaltenen Alkylreste sollen bis zu 7, bevorzugt bis zu 4 Kohlenstoffatome aufweisen. Im Falle der gesättigten Alkylreste sind die Formyl-, Acetyl-, Propionyl- und Butyrylgruppe als Substinent

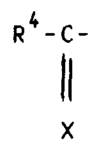

bzw. deren Hydroxyimino-Derivate bevorzugt. Im Falle der ungesättigten Kohlenwasserstoffreste sind $\alpha,\beta$-ungesättigte

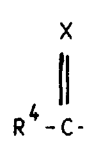

Gruppierungen, bei denen die C-Atome 2 und 3 der Kette die Doppelbindung tragen, bevorzugt.

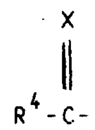

soll auch für einen Benzoylrest stehen.

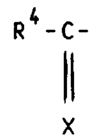

befindet sich bevorzugt in 3-oder 4-Stellung des Phenylrings.

Bevorzugte Verbindungen der allgemeinen Formel I sind:
17-Ethinyl-17β-hydroxy-11β-(4-acetylphenyl)-4,9,15-estratrien-3-on,
17-(Prop-1-inyl)-17β-hydroxy-11β-(4-acetylphenyl)-4,9,15-estratrien-3-on,
17-(Prop-2-inyl)-17β-hydroxy-11β-(4-acetylphenyl)-4,9,15-estratrien-3-on,
17-Ethinyl-17β-hydroxy-11β-(4-acetylphenyl)-18-methyl-4,9,15-estratrien-3-on
17-(Prop-1-inyl)-17β-hydroxy-11β-(4-acetylphenyl)-18-methyl-4,9,15-estratrien-3-on,
17-(Prop-2-inyl)-17β-hydroxy-11β-(4-acetylphenyl)-18-methyl-4,9,15-estratrien-3-on,
17-Methyl-17β-hydroxy-11β-(4-acetylphenyl)-4,9,15-estratrien-3-on,
17-Butyl-17β-hydroxy-11β-(4-acetylphenyl)-4,9,15-estratrien-3-on,
17-(3-Hydroxypropyl)-17β-hydroxy-11β-(4-acetylphenyl)-4,9,15-estratrien-3-on
17-(Prop-1-inyl)-17β-hydroxy-11β-(4-propionylphenyl)-4,9,15-estratrien-3-on,
17-(Prop-2-inyl)-17β-hydroxy-11β-(4-propionylphenyl)-4,9,15-estratrien-3-on,
17-Cyanomethyl-17β-hydroxy-11β-(4-acetylphenyl)-4,9,15-estratrien-3-on.

Die neuen 11β-Phenyl-4,9,15-estratriene der allgemeinen Formel I werden erfindungsgemäß ausgehend von Verbindungen der Formel II hergestellt.

Die Herstellung der Edukte der allgemeinen Formel II wiederum geht aus von 17-Keto-Steroiden der allgemeinen Formel III

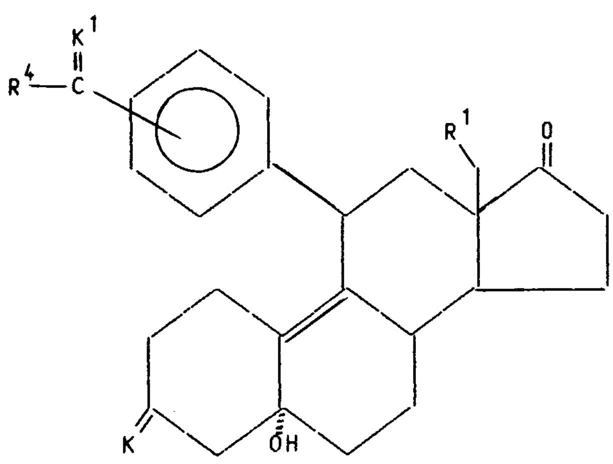

(III),

(siehe EP-A-190 759), worin K eine sauer hydrolysierbare Ketoschutzgruppe bedeutet, sowie $K^1$ für K in der genannten Bedeutung oder gemeinsam für ein Wasserstoffatom und eine geschützte Hydroxygruppe steht. Insbesondere stellt K eine in Form des Ketals, Thioketals, Oxims oder Methyloxims blockierte Ketogruppe dar.

Durch zum Beispiel modifizierte Saegusa-Oxidation [Tetrahedron 42 (1986)2971] der entsprechenden Enolverbindungen des 17-Ketons wird die C-15-Doppelbindung in den D-Ring eingeführt. Der hierzu beispielsweise benötigte Trimethylsilylenolether ist durch Überführung des 17-Ketons mit Lithiumdiisopropylamid in Tetrahydrofuran in das korrespondierende Enolat und Abfangen durch Trimethylchlorsilan darstellbar (Synthesis 1983, 1).

Die - nach Umwandlung der C-17-Ketogruppe in das C-17-Substitutionsmuster der letztlich gewünschten Bedeutung von $R^2$ und $R^3$ im Endprodukt der allgemeinen Formel I - erhaltenen Verbindungen der allgemeinen Formel II

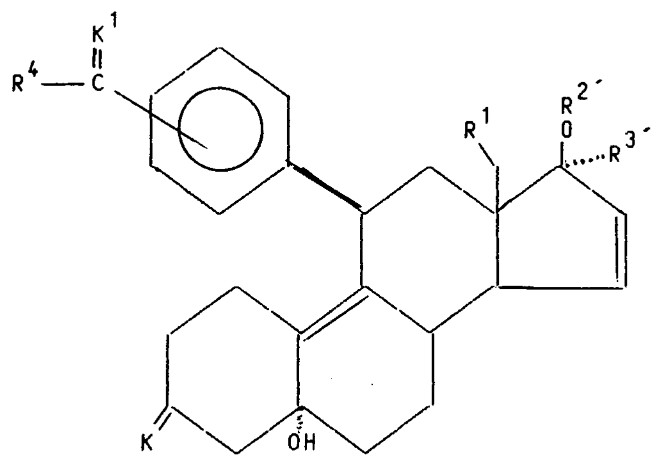

(II),

worin $R^1$, $R^4$, K und $K^1$ die oben angegebene Bedeutung haben, $R^{2'}$ und $R^{3'}$ die gleiche Bedeutung haben wie $R^2$ und $R^3$ , wobei vorhandene Hydroxy- und/oder Acyl-und/oder Alkingruppen gegebenenfalls geschützt sind, werden anschließend zur selektiven Wasserabspaltung unter Ausbildung der 4(5)-Doppelbindung und zur gleichzeitigen Entfernung vorhandener Schutzgruppen mit Säure oder einem sauren Ionenaustauscher behandelt.

Die saure Behandlung erfolgt in an sich bekannter Weise, indem man die Verbindung der Formel II in einem mit Wasser mischbaren Lösungsmittel, wie wäßrigem Methanol, Ethanol oder Aceton, löst und auf die Lösung katalytische Mengen Mineral-oder Sulfonsäure, wie Salzsäure, Schwefelsäure, Phosphorsäure, Perchlorsäure oder p-Toluolsulfonsäure, oder eine organische Säure, wie Essigsäure, so lange einwirken läßt, bis Wasser abgespalten ist und Schutzgruppen entfernt sind. Die Umsetzung, die bei Temperaturen von 0 °C bis 100 °C abläuft, kann auch mit einem sauren Ionenaustauscher vorgenommen werden. Der Verlauf der Umsetzung kann mit analytischen Methoden, beispielsweise durch Dünnschichtchromatographie entnommener Proben, verfolgt werden.

Sind außerdem nur im basischen Milieu abspaltbare Schutzgruppen vorhanden, wird vor oder nach der sauren Behandlung noch ein basisches Agens einwirken gelassen.

Die in den allgemeinen Formeln II und III von K, $R^{2'}$ und $R^{3'}$ umfaßten Schutzgruppen sind im sauren Milieu leicht abspaltbare Gruppen, z.B. die Ethylendioxyketal-, Ethylendithioketal-, 2,2-Dimethyltrimethylendioxyketal-, Hydroxyimino-, Methoxyimino-, Tetrahydropyranyl-, Methoxymethyl- oder Methoxyethylgruppe. Zum Schutz terminaler Acetylengruppen werden beispielsweise die Trimethylsilyl- oder tert.-Butyldimethylsilylgruppe verwendet, die im basischen Milieu abzuspalten sind.

Wird eine Verbindung der allgemeinen Formel II eingesetzt, deren $K^1$ eine geschützte Hydroxygruppe enthält, so wird diese anschließend mit einem der für die Oxidation allylischer Hydroxygruppen üblichen Oxidationsmittel, wie zum Beispiel Chromsäure, Pyridin, Pyridiniumdichromat, Pyridiniumchlorochromat, Braunstein, Silbercarbonat auf Celite, in die Oxofunktion überführt. Bevorzugt ist die bei Temperaturen zwischen -20 °C und +40 °C durchgeführte Umsetzung mit Braunstein.

Die so erhaltenen Verbindungen der allgemeinen Formel I mit X in der Bedeutung eines Sauerstoffatoms können gewünschtenfalls durch Umsetzung mit Hydroxylaminhydrochlorid in Gegenwart von tertiären Aminen bei Temperaturen zwischen -20 °C und +40 °C in die Oxime (Formel I mit X in der Bedeutung der Hydroxyiminogruppierung N OH, wobei die Hydroxygruppe syn- oder antiständig sein kann) überführt werden. Geeignete tertiäre Basen sind beispielsweise Trimethylamin, Triäthylamin, Pyridin, N,N-Dimethylaminopyridin, 1,5-Diazabicyclo[4.3.0]nonen-5 (DBN) und 1,5-Diazabicyclo[5.4.0]undecen-5 (DBU), wobei Pyridin bevorzugt ist.

Die Einführung der Substituenten $R^2$ und $R^3$ erfolgt nach den üblichen Verfahren des C-17-Seitenkettenaufbaus durch nucleophile Addition an das 17-Keton und Folgereaktionen ("Terpenoids and Stereoids", Specialist Periodical Report, The Chemical Society, London, Vol. 1-12).

Die nucleophile Addition von HC≡CU, in der U eine Schutzgruppe wie zum Beispiel Trimethylsilyl oder tert.-Butyldimethylsilyl oder Y bedeutet, erfolgt mit Hilfe einer Verbindung der allgemeinen Formel MC≡CU, in der U die oben angegebene Bedeutung hat und M ein Alkalimetall darstellt.
Die metallorganische Verbindung kann auch in situ gebildet und mit dem 17-Keton zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondere Dialkylether, Tetrahydrofuran, Dioxan, Benzol und Toluol geeignet.

Zur Herstellung der 17-Chlorethinylverbindung wird die metallorganische Chlorethinylverbindung in situ aus 1,2-Dichlorethylen und einer etherischen Alkalimetall-Lösung, wie z.B. Methyl-oder Butyllithiumlösung, gebildet und mit dem 17-Keton in Lösungsmitteln, wie Tetrahydrofuran oder Diethylether, umgesetzt.

17-Halogenethinylverbindungen können auch durch Halogenierung des entsprechenden Ethinyl-Edukts hergestellt werden (Angew. Chem. 96, 720 (1984)).

Die Einführung von 3-Hydroxypropin in 17-Stellung erfolgt durch Umsetzung des 17-Ketons mit dem Dianion des Propargylalkohols (3-Hydroxypropin), zum Beispiel dem in situ generierten Dikaliumsalz des Propargylalkohols, zum 17α-(3-Hydroxyprop-1-inyl)-17β-hydroxyderivat oder mit metallierten Derivaten des 3-Hydroxypropins, zum Beispiel mit 1-Lithium-3-(tetrahydropyran-2′-yloxy)-prop-1-in-1id, zum 17-[3-(Tetrahydropyran-2′-yloxy)-prop-1-inyl]-17β-hydroxyderivat.

Die Einführung der homologen Hydroxyalkin-Gruppen erfolgt in entsprechender Weise mit Homologen des Propargylalkohols.

Die Einführung von 3-Hydroxypropan in 17-Stellung erfolgt durch Umsetzung des 17-Ketons mit metallierten Derivaten von 3-Halogenpropanolen, wobei die Hydroxygruppe im Metallierungsschritt als Alkoholat (Tetrahedron Letters 1978, 3013) oder als geschützte Funktion vorliegt, zu der 17-(3-Hydroxypropyl)-17β-hydroxyverbindung bzw. zu der an der terminalen Hydroxygruppe geschützten Verbindung. Es kommen die gleichen Schutzgruppen, die oben genannt worden sind, infrage.

Die Einführung der homologen Hydroxyalkangruppen erfolgt in entsprechender Weise mit Homologen der 3-Halogenpropanole.

Der Aufbau der 17-Cyanmethylseitenkette erfolgt in an sich bekannter Weise aus dem 17-Keton zum Beispiel über eine Addition von $MCH_2CN$ mit M in der Bedeutung eines Alkalimetalls, bevorzugt Lithium.

Freie Hydroxygruppen in 17-Stellung und in den für $R^{3'}$ stehenden Resten können in an sich bekannter Weise verestert oder verethert werden.

Im übrigen wird auf die EP-A 0 190 759 verwiesen, in der zahlreiche Verbindungen mit den hier beanspruchten Substituenten beschrieben sind, mit der Abweichung, daß die dort beschriebenen Verbindungen keine $\Delta^{15}$-Doppelbindung besitzen.

Die neuen Verbindungen der allgemeinen Formel I sind wertvolle Pharmaka. So verfügen sie über eine starke Affinität zum Gestagenrezeptor, ohne selbst gestagene Aktivität zu besitzen. Sie sind kompetitive Antagonisten des Progesterons (Anti-Gestagene), da sie das zur Aufrechterhaltung der Schwangerschaft erforderliche Progesteron vom Rezeptor verdrängen. Sie sind zur postcoitalen Fertilitätskontrolle und zur Auslösung von Aborten geeignet.
Sie können auch gegen hormonelle Unregelmäßigkeiten, zur Menstruationsauslösung und zur Geburtseinleitung eingesetzt werden.

Außerdem können sie für die Behandlung von hormonabhängigen Carcinomen eingesetzt werden.
Die erfindungsgemäßen Verbindungen der allgemeinen Formel I weisen auch eine antiglucocorticoide Aktivität auf und können somit auch als Arzneimittel zur Therapie corticoid-induzierter Störungen (Glaukom) sowie zur Bekämpfung von Nebenwirkungen, die bei langfristiger Behandlung mit Glucocorticoiden auftreten (Cushing-Syndrom), eingesetzt werden. Sie ermöglichen daher auch die auf eine Supersekretion der Glucocorticoide zurückzuführenden Störungen, vor allem die Adipositas, Arteriosklerose, Hypertension, Osteoporose, Diabetes sowie die Insomnie zu bekämpfen.

Es wurde auch gefunden, daß die neuen Verbindungen der allgemeinen Formel I nicht nur sehr gute antigestagene und antiglucocorticoide Wirkungen zeigen, sondern daß bei ihnen auch eine Trennung beider Effekte zu beobachten ist.

Zur Kennzeichnung der antigestagenen Wirkung wurde die abortive Wirkung bestimmt.

Die Versuche wurden an weiblichen Ratten im Gewicht von ca. 200 g durchgeführt. Nach erfolgter Anpaarung wurde der Schwangerschaftsbeginn durch Nachweis von Spermien in Vaginalabstrichen gesichert. Der Tag des Spermiennachweises gilt als Tag 1 der Gravidität (= d1 p.c.).

Die Behandlung der Tiere mit der jeweils zu testenden Substanz bzw. dem Lösungsmittel erfolgte nach der Nidation der Blastocysten von d5 p.c. bis d7 p.c. An d9 p.c. wurden die Tiere getötet und die Uteri auf

Implantate und Resorptionsstellen hin untersucht. Von allen Uteri wurden Fotos angefertigt. Das Fehlen von Implantaten wurde als Abort gewertet.

Die Testsubstanzen wurden in einem Benzylbenzoat-Rizinusöl-Gemisch (Verhältnis 1:4) gelöst. Das Vehikelvolumen pro Einzeldosis betrug 0,2 ml. Die Behandlung erfolgte subcutan (s.c.).

Die Überlegenheit der erfindungsgemäßen Verbindungen soll durch Vergleich der biologischen Eigenschaften der erfindungsgemäßen Verbindungen

11β-(4-Acetylphenyl)-17β-hydroxy-17α-(prop-1-inyl)-4,9,15-estratrien-3-on (A),

11β-(4-Acetylphenyl)-17β-hydroxy-17α-(prop-2-inyl)-4,9,15-estratrien-3-on (B), dem in EP-A-0 057 115 beschriebenen

11β-(4-Dimethylaminophenyl)-17β-hydroxy-17α-(prop-1-inyl)-4,9,(10)-estradien-3 on RU 486 (C) und dem aus der EP-A-0 190 759 hervorgehenden

11β-(4-Acetylphenyl)-17β-hydroxy-17α-(prop-1-inyl)-4,9-estradien-3-on (D) gezeigt werden:

TABELLE 1

| ABORTIVTEST BEI DER GRAVIDEN RATTE | | |
|---|---|---|
| Substanz | Dosis mg/Tier/Tag s.c. | Abortrate n-Abort-positiv/n Gesamt |
| A | 3,0<br>1,0<br>0,3 | 4/4<br>4/4<br>4/4 |
| B | 3,0<br>1,0<br>0,3 | 4/4<br>4/4<br>4/4 |
| C | 3,0<br>1,0<br>0,3 | 4/4<br>2/4<br>0/4 |
| D | 3,0<br>1,0<br>0,3 | 4/4<br>4/4<br>4/4 |

Aus der Tabelle 1 ist zu entnehmen, daß die erfindungsgemäßen Verbindungen (A) und (B) bei einer Dosis von 0,3 mg abortiv voll wirksam sind, das heißt, sie sind um den Faktor 10 wirksamer als die bekannte Verbindung RU 486 (C), einer Substanz, die als Standard anzusehen ist (7th Int. Congress of Endocrinologiy July 1-7, 1984, Quebec City, Canada; Excerpta Medica, Amsterdam-OxfordPrinceton).

Es wurde auch die abortive Wirkung der erfindungsgemäßen Substanzen (A) und (B) unter Verwendung von Meerschweinchen als Versuchstiere bestimmt.

Dabei wurde gefunden, daß (A) und (B) überraschenderweise eine deutlich höhere abortive Wirksamkeit als die strukturell verwandte Verbindung (D) besitzen.

Testergebnisse die mit Meerschweinchen gewonnen wurden, lassen eine noch zuverlässigere Voraussage auf die beim Menschen zu erwartende abortive Wirksamkeit der getesteten Verbindung(en) zu, als dies die mit Ratten gewonnenen Daten gestatten.

Zur Kennzeichnung der antiglucocorticoiden Wirkung wurde der Einfluß der erfindungsgemäßen Substanzen auf die Tyrosin-Aminotransferase bestimmt. Das Test-System basiert auf einer Messung der Aktivität des Leberenzyms Tyrosin Aminotransferase (TAT) in Kulturen von RHC (Rat Hepatoma Cells) Zellen. Das Enzym katalysiert den ersten Schritt in der Verstoffwechselung von Tyrosin und ist sowohl in der Leber als auch in Hepatomzellen durch Glucocorticoide induzierbar. Die Aktivität ist in Rohextrakten leicht meßbar (Granner und Tomkins, (1970) Meth. Enzymol. 15, 633). Das Enzym überführt die Aminogruppe von Tyrosin auf 2-Oxoglutarsäure. Dabei entstehen Glutaminsäure und p-Hydroxy-phenylpyruvat. In alkalischer Lösung wird aus p-Hydroxyphenylpyruvat der stabilere p-Hydroxybenzaldehyd gebildet, dessen Absorbtion bei 331 nm gemessen wird. Die TAT-Aktivität in RHC-Zellen zeigt eine dosisabhängige Induktion mit Cortisol (max. Akt. bei $10^{-6}$ M) oder Dexamethason (max. Akt. bei $10^{-7}$ M). Die Aktivität läßt sich um den Faktor 4-6 über den Basalwert stimulieren. Gleichzeitige Behandlung mit Corticoid und Antiglucocorticoid führt zu einer Abnahame der TAT-Aktivität.

Die erfindungsgemäße Verbindung (A) zeigt in diesem Test 20-50% und die erfindungsgemäße Verbindung (B) weniger als 1% der Aktivität der Standardverbindung RU 486 (C).

Im Gestagen-Rezeptor-Bindungstest wird die Affinität der erfindungsgemäßen Verbindungen zum Gestagenrezeptor untersucht. Gemessen wird dabei die Verdrängung des Agonisten durch den Antagonisten.

Man verwendet Cytosol aus Kaninchenuterushomogenat, das das Rezeptormolekül - ein Protein - enthält. Dieses bindet mit hoher Affinität und geringer Kapazität Progesteron. Wenn diese Rezeptoren mit $^{3}H$-Progesteron in Gegenwart der zu prüfenden, unmarkierten Substanz beladen werden, so hängt es von der Konzentration und von der Bindungsaffinität der zu untersuchenden Verbindung ab, wie stark $^{3}H$-Progesteron vom Rezeptor verdrängt wird. Nach Trennung des Rezeptor-gebundenen Progesterons vom nichtgebundenen kann man die Bindung in Prozent ermitteln und diesen Wert gegen den Logarithmus der molaren Konzentration der Prüfsubstanz auftragen. Man erhält charakteristische dosisabhängige Verdrängungskurven und kann nun die Konzentration der Prüfsubstanz ermitteln, die erforderlich ist, um die Referenzsubstanz vollständig vom Rezeptor zu verdrängen. Der Kompetitionsfaktor K als Maß für die Bindungsstärke ist definiert als das Verhältnis der Konzentration der Prüfsubstanz zur Konzentration der Referenzsubstanz (Progesteron), bei der beide Verbindungen eine gleich große Verdrängung von $^{3}H$-Progesteron vom Progesteron-Rezeptorkomplex zeigen, so daß ein niedriger K-Wert große Bindungsstärke (hohe Affinität) anzeigt.

TABELLE 2

| **GESTAGEN-REZEPTOR-BINDUNGSTEST** | |
|---|---|
| Verbindung | Kaninchenuterus K (gestagen) |
| A | 1,9 |
| B | 2,9 |
| C | 2,9 |
| D | 1,0 |

Die Tabelle zeigt, daß von den stellvertretend genannten erfindungsgemäßen Verbindungen (A) und (B) die Verbindung (A) im Gestagen-Rezeptor-Bindungstest wesentlich stärker wirksam als und die Verbindung (B) ungefähr gleich stark wirksam wie die Standardverbindung (C) ist.

Zusammenfassend kann also festgestellt werden, daß die erfindungsgemäßen Verbindungen sowohl untereinander als auch gegenüber der Standardverbindung (C) RU486 und der strukturell ähnlichen Verbindung (D) eine deutliche Dissoziation der antiglucocorticoiden und antigestagenen Eigenschaften aufweisen.

Eine weitere hervorstechende Eigenschaft der erfindungsgemäßen Verbindungen ist ihre im Vergleich zu den Verbindungen des Standes der Technik hohe metabolische Stabilität

Die Erfindung betrifft somit auch Arzneimittel auf Basis der pharmazeutisch verträglichen, d. h. in den verwendeten Dosen nicht toxischen Verbindungen der allgemeinen Formel I und gegebenenfalls der üblichen Hilfs- und Trägerstoffe.

Die erfindungsgemäßen Verbindungen können nach an sich bekannten Methoden der Galenik zu pharmazeutischen Präparaten für die enterale, perkutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.

Der oder die Wirkstoffe können dabei mit den in der Galenik üblichen Hilfsstoffen wie z. B. Gummiarabikum, Talk, Stärke, Mannit, Methylcellulose, Laktose, Tensiden wie Tweens® oder Myrj®, Magnesiumstearat, wäßrigen oder nicht-wäßrigen Trägern, Paraffinderivaten, Netz-, Dispergier-, Emulgier-, Konservierungsmitteln und Aromastoffen zur Geschmackskorrektur (z.B. ätherischen Ölen) gemischt werden.

Die Erfindung betrifft somit auch pharmazeutische Zusammensetzungen, die als Wirkstoff zumindest eine erfindungsgemäße Verbindung enthalten.

Eine Dosiseinheit enthält etwa 1 - 100 mg Wirkstoff(e). Die Dosierung der erfindungsgemäßen Verbindungen liegt beim Menschen bei etwa 1 - 1000 mg pro Tag.

**BEISPIEL 1**

17-(Prop-1-inyl)-17β-hydroxy-11β-(4-acetylphenyl)-4,19,15-estratrien-3-on

6,1 g 17-(Prop-1-inyl)-11β-{4-[1,1-(2,2-dimethyltrimethylendioxy)-ethyl]-phenyl}3,3-(2,2-dimethyltrimethylendioxy)-9,15-estradien-5α,17β-diol werden in 100 ml 70%iger wäßriger Essigsäure gelöst und 2,5 Stunden bei 50°C unter Schutzgas gerührt. Nach dem Abkühlen gießt man in Eiswasser, neutralisiert durch Zugabe von wäßriger Ammoniaklösung und extrahiert mit Methylenchlorid. Die vereinigten organischen Phasen werden über Natriumsulfat getrocknet und am Vakuum eingeengt. Den Rückstand chromatographiert man mit einem Gemisch aus Hexan/Essigester an Kieselgel. Es werden 3,5 g der Titelverbindung als weißer Schaum isoliert.
Kristallisation aus Essigester/Aceton führt zu 3,25 g der Titelverbindung.
Schmelzpunkt: 162-164°C ; $[\alpha]_D^{20}$ = 14,8° ($CHCl_3$; c=0,505).

Die Herstellung des Ausgangsmaterials erfolgt auf folgendem Wege:

a) Unter Schutzgas werden 10 ml Diisopropylamin in 290 ml absolutem Tetrahydrofuran bei -10°C vorgelegt und mit 50 ml einer 1,6 m n-Butyllithiumlösung (Hexan) versetzt. Es wird eine halbe Stunde bei 0°C nachgerührt, dann wieder auf -10°C gekühlt und 13,6 g 11β-{4-[1,1-(2,2-dimethyltrimethylendioxy)-ethyl]-phenyl}-5α-hydroxy-3,3-(2,2-dimethyltrimethylendioxy)-9-estren-17-on (Herstellung nach Europäische Patentanmeldung 86101548.5, Publikations-Nr. 190759, Beispiel 6) gelöst in 150 ml absolutem Tetrahydrofuran zugetropft. Nach erfolgter Zugabe rührt man 15 Minuten nach und tropft dann 17,2 ml Trimethylchlorsilan zu. Anschließend wird das Reaktionsgemisch auf eiskalte gesättigte Natriumhydrogencarbonatlösung gegossen und die wäßrige Phase mit Essigester extrahiert. Die vereinigten organischen Phasen werden mehrmals mit gesättigter Ammoniumchloridlösung gewaschen und am Vakuum eingeengt. Den Rückstand kristallisiert man aus 50 ml Acetonitril. Es werden 13,2 g 17-Trimethylsilyloxy-11β-{4-[1,1-(2,2-dimethyltrimethylendioxy)-ethyl]-phenyl}-3,3-(2,2-dimethyltrimethylendioxy)-9,16-estradien-5α-ol erhalten.
$^1$H-NMR ($CD_2Cl_2$) δ : 4,47 ppm (1H,m,H-16); 4,27 (1H,d J=7,5 Hz,H-11); 1,48 ppm (3H,s, H-$CH_3$ ) 1,21 (3H,s, H-$CH_3$ ); 1,01 ppm (3H,s, H-$CH_3$ ) 0,83 (3H,s, H-$CH_3$ ); 0,54 ppm (3H,s, H-$CH_3$ ) 0,5 (3H,s,H-18); 0,15 ppm (9H,s,3 x H-$CH_3$ Si ).

b) In 150 ml absolutem Acetonitril werden 4,27 g Palladium(II)-acetat vorgelegt und mit 12,12 g der unter a) hergestellten Verbindung versetzt. Das Reaktionsgemisch wird 16 Stunden bei Raumtemperatur gerührt, anschließend über Kieselgel filtriert und der Filterrückstand gut mit Methylenchlorid gewaschen. Die organische Phase wird am Vakuum eingeengt und der Rückstand an Kieselgel chromatographiert. Es werden 10 g 11β-{4-[1,1-(2,2-dimethyltrimethylendioxy)-ethyl]-phenyl}-5α-hydroxy-3,3-(2,2-dimethyltrimethylendioxy)-9,15-estradien-17-on als weißer Schaum isoliert.
$^1$H-NMR ($CDCl_3$) δ : 7,57 ppm (1H,d J=5,5 Hz,H-15); 7,18-7,37ppm (4H,m,H-aromatisch) 6,03 ppm (1H,m,H-16); 4,38 ppm(1H,d J=7,5 Hz,H-11); 1,52ppm (3H,s,H$CH_3$); 1,25 ppm(3H,s,H-$CH_3$); 1,04ppm (3H,s,H-$CH_3$); 0,86 ppm (3H,s,H-$CH_3$); 0,75ppm-(3H,s,H-$CH_3$); 0,55ppm (3H,s,H-18).

c) 500 ml absolutes Tetrahydrofuran werden durch 30 minütiges Einleiten von Methylacetylen bei 0°C gesättigt. Anschließend tropft man bei 0 bis 5°C 42,2 ml einer 1,6 m Lösung von n-Butyllithium in Hexan zu, rührt nach Zugabe 15 Minuten nach und gibt dann eine Lösung von 9,5 g der unter b) hergestellten Verbindung in 50 ml absolutem Tetrahydrofuran tropfenweise hinzu. Das Reaktionsgemisch wird nach erfolgter Zugabe 60 Minuten nachgerührt, auf Eiswasser gegossen und die wäßrige Phase mit Essigester extrahiert. Die vereinigten organischen Phasen engt man am Vakuum ein, nachdem man sie über Natriumsulfat getrocknet hatte. Der Rückstand wird an Aluminiumoxid (neutral, Stufe III) mit einem Gemisch aus Essigester/Hexan chromatographiert. Es werden 9,6 g 17-(Prop-1-inyl)-11β-{4-[1,1-(2,2-dimethyltrimethylendioxy)-ethyl]-phenyl}-3,3-(2,2-dimethyltrimethylendioxy)-9,15-estradien-5α, 17β-diol als weißer Schaum erhalten.
$^1$H-NMR ($CDCl_3$) δ : 7,2-7,4 ppm (4H,m,H-aromatisch); 5,95 ppm(1H,d J=6HZ,H-15); 5,7 ppm (1H,dd J=6 und J=2 Hz,H-16); 4,4 ppm (1H,d breit J=8 Hz,H-11); 1,92 ppm (3H,s,H-$CH_3$-C≡C-); 1,52 ppm (3H,s,H-$CH_3$); 1,25 ppm (3H,s,H-$CH_3$); 1,03 ppm(3H,-s,H-$CH_3$); 0,88 ppm (3H,s,H-$CH_3$); 0,56 ppm (3H,s,H-$CH_3$); 0,52 ppm (3H,s,H 18).

**BEISPIEL 2**

17-(Prop-2-inyl)-17β-hydroxy-11β-(4-acetylphenyl)-4,9,15-estratrien-3-on

350 mg 17-(3-Trimethylsilylprop-2-inyl)-17β-hydroxy-11β-(4-acetylphenyl)-4,9,-15-estratrien-3-on werden in Methanol gelöst und nach Zugabe von 387 mg Kaliumcarbonat 1.5 Stunden bei 23 °C gerührt. Man gießt auf Wasser und extrahiert mit Essigester. Die vereinigten organischen Phasen werden über Natriumsulfat getrocknet und am Vakuum eingeengt. Den Rückstand chromatographiert man mit einem Gemisch aus Hexan/Essigester an Kieselgel. Es werden 205 mg der Titelverbindung als weißer Schaum isoliert. Kristallisation aus Ether führt zu 176 mg der Titelverbindung.
Schmelzpunkt: 152-154 °C; $[\alpha]_D^{20}$ = 156,8 ° ($CHCl_3$; c = 0,500)

Die Herstellung des Ausgangsmaterials erfolgt auf folgendem Wege:

a) Unter Schutzgas werden 1,17 g 1-(Trimethylsilyl)-1-propin in 500 ml absolutem Tetrahydrofuran bei -5 °C vorgelegt und mit 6,8 ml einer 1,6 m n-Butyllithiumlösung (Hexan) versetzt. Es wird eine Stunde bei dieser Temperatur nachgerührt, dann auf -78 °C gekühlt und 1,5 g der unter Beispiel 1b) hergestellten Verbindung gelöst in 200 ml Tetrahydrofuran zugetropft. Nach erfolgter Zugabe rührt man 15 Stunden bei 23 °C nach. Anschließend wird auf kalte gesättigte Ammoniumchloridlösung gegossen und die wäßrige Phase mit Essigester extrahiert. Die vereinigten organischen Phasen werden erst mit gesättigter Natriumhydrogencarbonatlösung dann mit gesättigter Kochsalzlösung gewaschen und am Vakuum eingeengt. Den Rückstand chromatographiert man mit einem Gemisch aus Hexan/Essigester an neutralem Aluminiumoxid (Aktivität 3). Es werden 1,22 g 17-(3-Trimethylsilylprop-2-inyl)-11β-{4-[1,1-(2,2-dimethyltrimethylendioxy)-ethyl]-phenyl}-3,3-(2,2-dimethyltrimethylendioxy)-9,15-estradien-5 α,17β-diol erhalten.
$^1$H-NMR($CD_2Cl_2$)δ: 7.23 ppm, 7.29 ppm (4H,AA'BB'-System, J = 9 HZ,H-aromatisch); 5.93 ppm (1H,d J = 6 Hz, H-16); 5.69 ppm (1H,dd J = 6 und 4Hz,H-15); 4.38 ppm (1H,d breit J = 8 Hz, H-11); 4.27 ppm (1H,s,OH); 2.45 ppm (2H,s,$CH_2$-C≡C-); 1.49 ppm (3H,s,H-$CH_3$); 1.22 ppm (3H,s,H-$CH_3$); 1.04 ppm (3H,s, H-$CH_3$); 0,86 ppm (3H,s,H-$CH_3$); 0.58 ppm (6H,s,H-18 und H-$CH_3$); 0.18 ppm (9H,s,3xH-$CH_3$Si).

b) 1,2 g der unter a) hergestellten Verbindung werden in 15 ml 70%iger wäßriger Essigsäure gelöst und 15 Minuten bei 50 °C unter Schutzgas gerührt. Nach dem Abkühlen gießt man in gesättigte Natriumhydrogencarbonatlösung und extrahiert mit Essigester. Die vereinigten organischen Phasen werden über Natriumsulfat getrocknet und am Vakuum eingeengt. Den Rückstand chromatographiert man mit einem Gemisch aus Hexan/Essigester an Kieselgel. Nach Kristallisation aus Ether werden 582 mg 17-(3-Trimethylsilylprop-2-inyl)-17β-hydroxy-11β-(4-acetylphenyl)-4,9,15-estratrien-3-on als weiße Kristalle erhalten.
Schlemzpunkt: 186-189 °C; $[\alpha]_D^{20}$ = 123,6 ° ($CHCl_3$; c = 0.500)

**Patentansprüche**

1. 11β-Phenyl-4,9,15-estratriene der allgemeinen Formel I

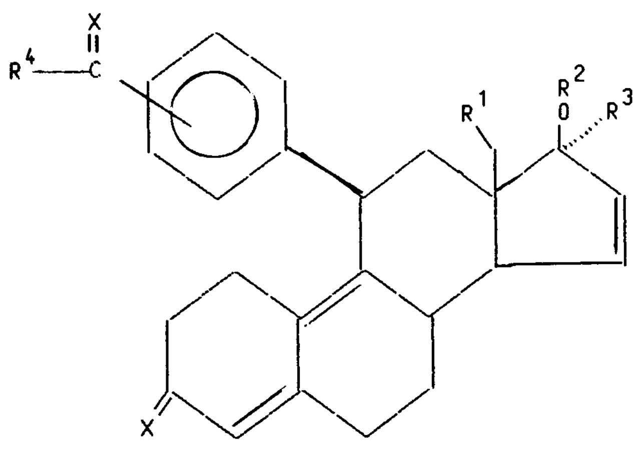

(I),

worin

X für ein Sauerstoffatom oder eine Hydroxyiminogruppierung N~OH,

$R^1$ für ein Wasserstoffatom oder eine Methylgruppe,
$R^2$ für ein Wasserstoffatom, einen Alkylrest oder einen Acylrest mit jeweils 1 bis 10 Kohlenstoffatomen,
$R^3$ für ein Wasserstoffatom, eine Cyanmethylgruppe, oder den Rest $-(CH_2)_nCH_2Z$, wobei n die Ziffern 0, 1, 2, 3, 4 oder 5 bedeutet und Z = -H oder $-OR^5$ ist mit $R^5$ gleich ein Wasserstoffatom oder ein Alkyl- oder Acylrest mit jeweils 1 bis 10 Kohlenstoffatomen, oder $R^3$ für den Rest $-(CH_2)_mC{\equiv}C\text{-}Y$, wobei m = 0,1 oder 2 und Y ein Wasserstoff-, Chlor-, Fluor-, Jod- oder Bromatom, eine Alkyl-, Hydroxyalkyl-, Alkoxyalkyl- oder Acyloxyalkylgruppe mit jeweils 1 bis 10 Kohlenstoffatomen bedeutet,
$R^4$ für einen geradkettigen oder verzweigten, gesättigten oder ungesättigten Alkylrest mit bis zu 7 Kohlenstoffatomen oder für einen Phenylrest

stehen.

**2.** 17-Ethinyl-17β-hydroxy-11β-(4-acetylphenyl)-4,9,15-estratrien-3-on,
17-(Prop-1-inyl)-17β-hydroxy-11β-(4-acetylphenyl)-4,9,15-estratrien-3-on,
17-(Prop-2-inyl)-17β-hydroxy-11β-(4-acetylphenyl)-4,9,15-estratrien-3-on,
17-Ethinyl-17β-hydroxy-11β-(4-acetylphenyl)-18-methyl-4,9,15-estratrien-3-on
17-(Prop-1-inyl)-17β-hydroxy-11β-(4-acetylphenyl)-18-methyl-4,9,15-estratrien-3-on,
17-(Prop-2-inyl)-17β-hydroxy-11β-(4-acetylphenyl)-18-methyl-4,9,15-estratrien-3-on,
17-Methyl-17β-hydroxy-11β-(4-acetylphenyl)-4,9,15-estratrien-3-on,
17-Butyl-17β-hydroxy-11β-(4-acetylphenyl)-4,9,15-estratrien-3-on,
17-(3-Hydroxypropyl)-17β-hydroxy-11β-(4-acetylphenyl)-4,9,15-estratrien-3-on
17-(Prop-1-inyl)-17β-hydroxy-11β-(4-propionylphenyl)-4,9,15-estratrien-3-on,
17-(Prop-2-inyl)-17β-hydroxy-11β-(4-propionylphenyl)-4,9,15-estratrien-3-on,
17-Cyanomethyl-17β-hydroxy-11β-(4-acetylphenyl)-4,9,15-estratrien-3-on.

**3.** Verfahren zur Herstellung von Verbindungen der allgemeinen Formel I

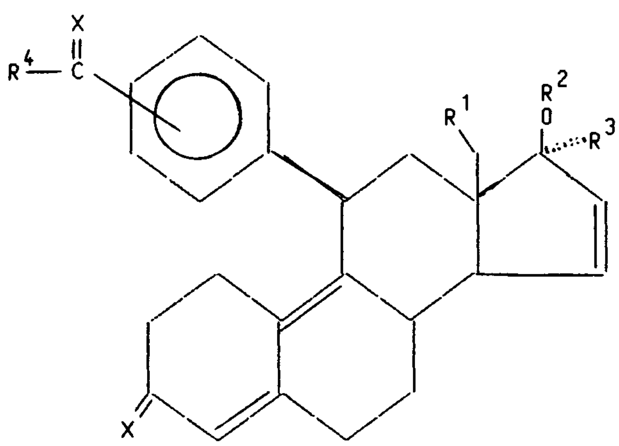

(I),

worin
X für ein Sauerstoffatom oder eine Hydroxyiminogruppierung N~OH,
$R^1$ für ein Wasserstoffatom oder eine Methylgruppe,
$R^2$ für ein Wasserstoffatom, einen Alkylrest oder einen Acylrest mit jeweils 1 bis 10 Kohlenstoffatomen,
$R^3$ für ein Wasserstoffatom, eine Cyanmethylgruppe, oder den Rest $-(CH_2)_nCH_2Z$, wobei n die Ziffern 0, 1, 2, 3, 4 oder 5 bedeutet und Z = -H oder $-OR^5$ ist mit $R^5$ gleich ein Wasserstoffatom oder ein Alkyl- oder Acylrest mit jeweils 1 bis 10 Kohlenstoffatomen, oder $R^3$ für den Rest $-(CH_2)_mC{\equiv}C\text{-}Y$, wobei m = 0,1 oder 2 und Y ein Wasserstoff-, Chlor-, Fluor-, Jod-oder Bromatom, eine Alkyl-, Hydroxyalkyl-, Alkoxyalkyl- oder Acyloxyalkylgruppe mit jeweils 1 bis 10 Kohlenstoffatomen bedeutet,
$R^4$ für einen geradkettigen oder verzweigten, gesättigten oder ungesättigten Alkylrest mit bis zu 7 Kohlenstoffatomen, oder für einen Phenylrest

stehen,

dadurch gekennzeichnet, daß man in an sich bekannter Weise eine Verbindung der allgemeinen Formel II

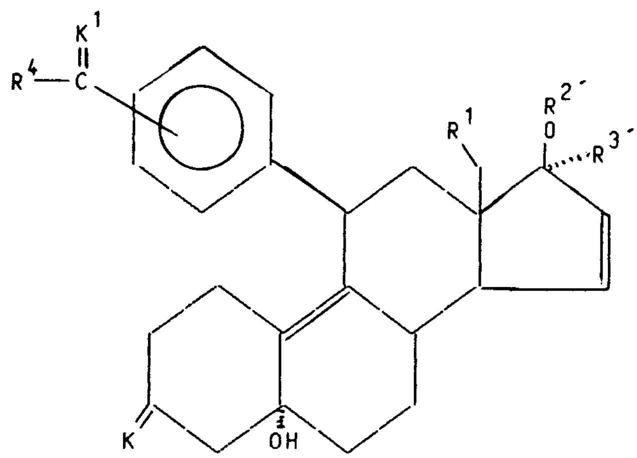

(II),

worin $R^1$ und $R^4$ die oben angegebene Bedeutung haben, K eine sauer hydrolisierbare Ketoschutzgruppe bedeutet, $R^{2'}$ und $R^{3'}$ die gleiche Bedeutung haben wie $R^2$ und $R^3$, wobei vorhandene Hydroxy- und/oder Acyl- und/oder terminale Alkingruppen gegebenenfalls geschützt sind, sowie $K^1$ für K in der genannten Bedeutung oder gemeinsam für ein Wasserstoffatom und eine geschützte Hydroxygruppe steht, der Einwirkung eines sauren Agens, das zur Freisetzung geschützter Funktion(en) und zur selektiven Abspaltung der 5α-Hydroxygruppe unter gleichzeitiger Ausbildung der 4(5)Doppelbindung befähigt ist, sowie bei Anwesenheit nur im basischen Milieu abspaltbarer Schutzgruppen vor oder nach der Einwirkung des sauren Agens, der Einwirkung eines basischen Agens unterwirft, eine in $K^1$ gegebenenfalls enthaltene Hydroxygruppe oxidiert, und gegebenenfalls vorhandene freie Hydroxygruppen in 17-Stellung und/oder in $R^{3'}$ gewünschtenfalls verestert oder verethert und gewünschtenfalls anschließend das Produkt der allgemeinen Formel I mit X in der Bedeutung eines Sauerstoffatoms mit Hydroxylamin-hydrochlorid in Gegenwart von tertiären Aminen umsetzt.

**4.** Pharmazeutische Präparate, gekennzeichnet durch einen Gehalt an Verbindungen gemäß den Ansprüchen 1 und 2.

**5.** Verwendung von Verbindungen gemäß den Ansprüchen 1 und 2 zur Herstellung von Arzneimitteln.

## Claims

**1.** 11β-phenyl-4,9,15-oestratrienes of the general formula I

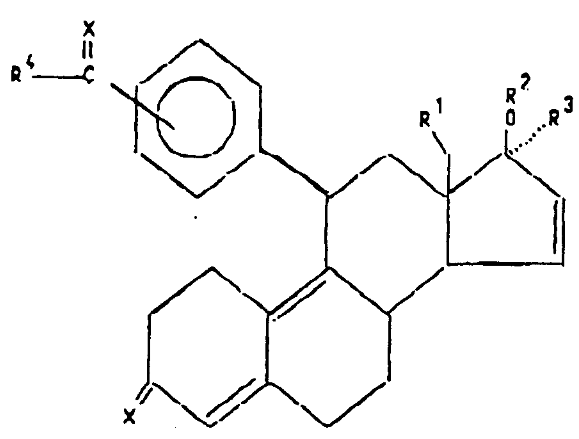

(I)

wherein

X represents an oxygen atom or a hydroxyimino grouping N~OH,

$R^1$ represents a hydrogen atom or a methyl group,

$R^2$ represents a hydrogen atom, or an alkyl radical or an acyl radical each of which has from 1 to 10 carbon atoms,

$R^3$ represents a hydrogen atom, a cyanomethyl group, or the radical $-(CH_2)_nCH_2Z$, wherein n represents the number 0, 1, 2, 3, 4 or 5 and Z is -H or $OR^5$, $R^5$ being a hydrogen atom or an alkyl or acyl radical each of which has from 1 to 10 carbon atoms, or $R^3$ represents the radical $-(CH_2)_mC{\equiv}C\text{-}Y$, wherein m is 0, 1 or 2 and Y represents a hydrogen, chlorine, fluorine, iodine or bromine atom or an alkyl, hydroxyalkyl, alkoxyalkyl or acyloxyalkyl group each of which has from 1 to 10 carbon atoms,

$R^4$ represents a straight-chain or branched, saturated or unsaturated alkyl radical having up to 7 carbon atoms, or represents a phenyl radical.

**2.** 17-ethynyl-17β-hydroxy-11β-(4-acetylphenyl)-4,9,15-oestratrien-3-one,
17-(prop-1-ynyl)-17β-hydroxy-11β-(4-acetylphenyl)-4,9,15-oestratrien-3-one,
17-(prop-2-ynyl)-17β-hydroxy-11β-(4-acetylphenyl)-4,9,15-oestratrien-3-one,
17-ethynyl-17β-hydroxy-11β-(4-acetylphenyl)-18-methyl-4,9,15-oestratrien-3-one,
17-(prop-1-ynyl)-17β-hydroxy-11β-(4-acetylphenyl)-18-methyl-4,9,15-oestratrien-3-one,
17-(prop-2-ynyl)-17β-hydroxy-11β-(4-acetylphenyl)-18-methyl-4,9,15-oestratrien-3-one,
17-methyl-17β-hydroxy-11β-(4-acetylphenyl)-4,9,15-oestratrien-3-one,
17-butyl-17β-hydroxy-11β-(4-acetylphenyl)-4,9,15-oestratrien-3-one,
17-(3-hydroxypropyl)-17β-hydroxy-11β-(4-acetylphenyl)-4,9,15-oestratrien-3-one,
17-(prop-1-ynyl)-17β-hydroxy-11β-(4-propionylphenyl)-4,9,15-oestratrien-3-one,
17-(prop-2-ynyl)-17β-hydroxy-11β-(4-propionylphenyl)-4,9,15-oestratrien-3-one,
17-cyanomethyl-17β-hydroxy-11β-(4-acetylphenyl)-4,9,15-oestratrien-3-one.

**3.** Process for the preparation of compounds of the general formula I

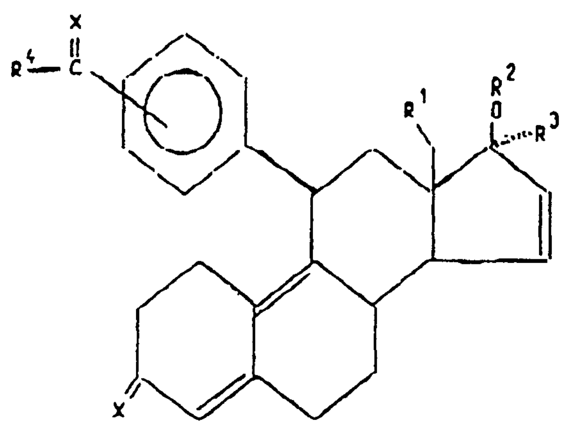

(I)

wherein

X represents an oxygen atom or a hydroxyimlno grouping N~OH,

$R^1$ represents a hydrogen atom or a methyl group,

$R^2$ represents a hydrogen atom, or an alkyl radical or an acyl radical each of which has from 1 to 10 carbon atoms,

$R^3$ represents a hydrogen atom, a cyanomethyl group, or the radical $-(CH_2)_nCH_2Z$, wherein n represents the number 0, 1, 2, 3, 4 or 5 and Z is -H or $-OR^5$, $R^5$ being a hydrogen atom or an alkyl or acyl radical each of which has from 1 to 10 carbon atoms, or $R^3$ represents the radical $-(CH_2)_mC{\equiv}C\text{-}Y$, wherein m is 0, 1 or 2 and Y represents a hydrogen, chlorine, fluorine, iodine or bromine atom or an alkyl, hydroxyalkyl, alkoxyalkyl or acyloxyalkyl group each of which has from 1 to 10 carbon atoms,

$R^4$ represents a straight-chain or branched, saturated or unsaturated alkyl radical having up to 7 carbon atoms, or represents a phenyl radical,

characterised in that a compound of the general formula II

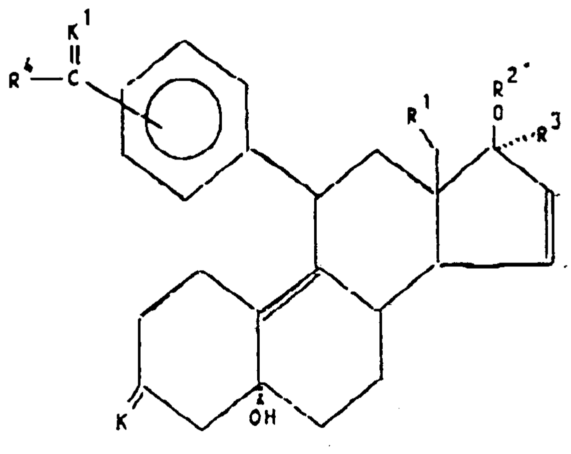

(II)

wherein $R^1$ and $R^4$ are as defined above, K is an acidically hydrolysable keto protecting group, $R^{2'}$ and $R^{3'}$ are as defined for $R^2$ and $R^3$, any hydroxy and/or acyl and/or terminal alkyne groups present optionally being protected, and $K^1$ is as defined for K or represents a hydrogen atom and a protected hydroxy group jointly, is subjected in a manner known per se to the action of an acidic agent that is capable of freeing protected function(s) and of selectively removing the 5α-hydroxy group to form, simultaneously, the 4(5)-double bond, and, in the case of the presence of protecting groups that can be removed only in a basic medium, is subjected, before or after the action of the acidic agent, to the action of a basic agent, a hydroxy group that may be contained in $K^1$ is oxidised, and any free hydroxy groups present in the 17-position and/or in $R^{3'}$ are, if desired, esterified or etherified and, if desired, the product of the general formula I wherein X represents an oxygen atom is then reacted with hydroxylamine hydrochloride in the presence of tertiary amines.

**4.** Pharmaceutical compositions characterised by a content of compounds according to claims 1 and 2.

**5.** The use of compounds according to claims 1 and 2 in the preparation of medicaments.

## Revendications

**1.** 11β-Phényl-4,9,15-estratriènes de formule générale I ci-dessus

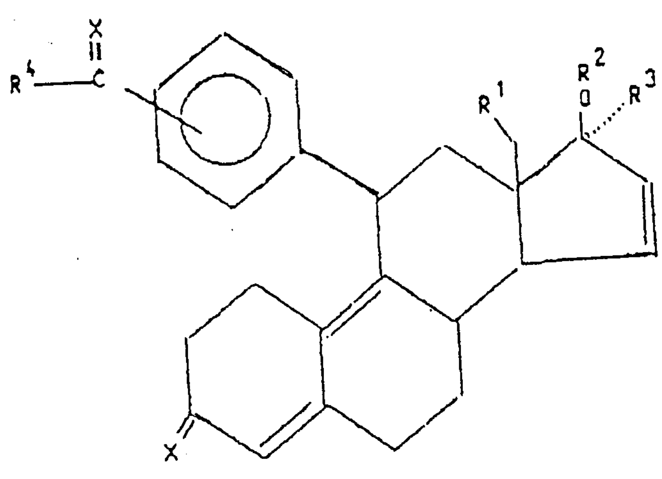

(I),

dans laquelle

X    représente un atome d'oxygène ou un groupe hydroxy-imino N~OH,

$R^1$ un atome d'hydrogène ou le groupe méthyle,
$R^2$ un atome d'hydrogène ou un alkyle ou un acyle pouvant avoir chacun de 1 à 10 atomes de carbone,
$R^3$ un atome d'hydrogène, un groupe cyanométhyle ou un radical $-(CH_2)_nCH_2Z$, n étant le nombre 0, 1, 2, 3, 4 ou 5 et Z un atome d'hydrogène ou un groupe $-OR^5$ et $R^5$ un atome d'hydrogène ou un alkyle ou un acyle pouvant avoir chacun de 1 à 10 atomes de carbone, ou bien $R^3$ désigne un radical $-(CH_2)_mC{\equiv}C-Y$, m étant le nombre 0, 1 ou 2 et Y un atome d'hydrogène, de chlore, de fluor, d'iode ou de brome ou encore un alkyle, un hydroxyalkyle, un alcoxyalkyle ou un acyloxyalkyle pouvant avoir chacun de 1 à 10 atomes de carbone, et
$R^4$ représente un alkyle à chaîne linéaire ou ramifiée, saturée ou insaturée, pouvant avoir jusqu'à 7 atomes de carbones, ou bien un radical phényle.

**2.** 17-Ethynyl-17β-hydroxy-11β-(4-acétylphényl)-4,9,15-estratriène-3-one,
17-(Prop-1-ynyl)-17β-hydroxy-11β-(4-acétylphényl)-4,9,15-estratriène-3-one,
17-(Prop-2-ynyl)-17β-hydroxy-11β-(4-acétylphényl)-4,9,15-estratriène-3-one,
17-Ethynyl-17β-hydroxy-11β-(4-acétylphényl)-18-méthyl-4,9,15-estratriène-3-one,
17-(Prop-1-ynyl)-17β-hydroxy-11β-(4-acétylphényl)-18-méthyl-4,9,15-estratriène-3-one,
17-(Prop-2-ynyl)-17β-hydroxy-11β-(4-acétylphényl)-18-méthyl-4,9,15-estratriène-3-one,
17-Méthyl-17β-hydroxy-11β-(4-acétylphényl)-4,9,15-estratriène-3-one,
17-Butyl-17β-hydroxy-11β-(4-acétylphényl)-4,9,15-estratriène-3-one,
17-(3-Hydroxypropyl)-17β-hydroxy-11β-(4-acétylphényl)-4,9,15-estratriène-3-one,
17-(Prop-1-ynyl)-17β-hydroxy-11β-(4-proprionylphényl)-4,9,15-estratriène-3-one,
17-(Prop-2-ynyl)-17β-hydroxy-11β-(4-propionylphényl)-4,9,15-estratriène-3-one,
17-Cyanométhyl-17β-hydroxy-11β-(4-acétylphényl)-4,9,15-estratriène-3-one.

**3.** Procédé de préparation de composés de formule générale I

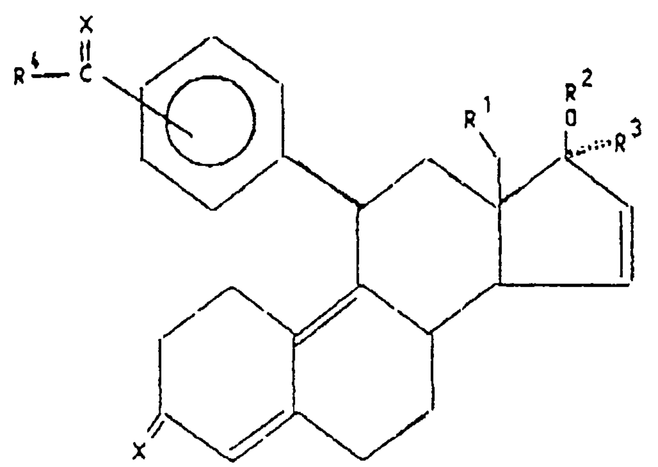

(I),

dans laquelle
X représente un atome d'oxygène ou un groupe hydroxy-imino N~OH,
$R^1$ un atome d'hydrogène ou le groupe méthyle,
$R^2$ un atome d'hydrogène ou un alkyle ou un acyle pouvant avoir chacun de 1 à 10 atomes de carbone,
$R^3$ un atome d'hydrogène, un groupe cyanométhyle ou un radical $-(CH_2)_nCH_2Z$, n étant le nombre 0, 1, 2, 3, 4 ou 5 et Z un atome d'hydrogène ou un groupe $-OR^5$ et $R^5$ un atome d'hydrogène ou un alkyle ou un acyle pouvant avoir chacun de 1 à 10 atomes de carbone, ou bien $R^3$ désigne un radical $-(CH_2)mC{\equiv}C-Y$, m étant le nombre 0, 1 ou 2 et Y un atome d'hydrogène, de chlore, de fluor, d'iode ou de brome ou encore un alkyle, un hydroxyalkyle, un alcoxyalkyle ou un acyloxyalkyle pouvant avoir chacun de 1 à 10 atomes de carbone, et

$R^4$ représente un alkyle à chaîne linéaire ou ramifiée, saturée ou insaturée, pouvant avoir jusqu'à 7 atomes de carbones, ou bien un radical phényle,

procédé caractérisé en ce que l'on soumet d'une manière en elle-même connue un composé de formule générale II

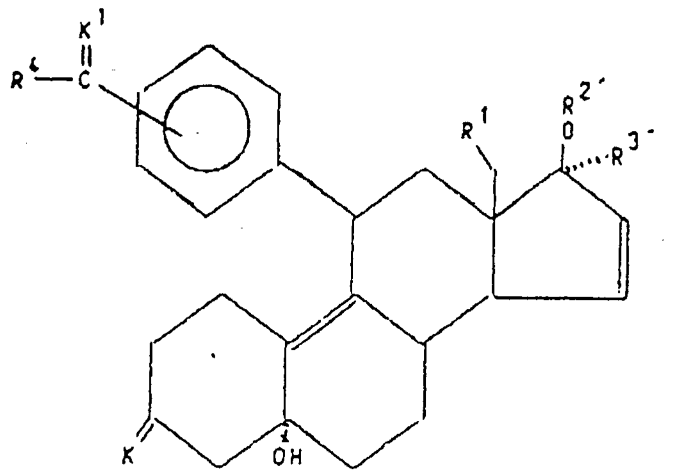

(II),

(dans laquelle $R^1$ et $R^4$ ont les mêmes significations que dans la formule I, K désigne un groupe protecteur de céto hydrolysable en milieu acide et $R^2$ et $R^3$ ont également les mêmes significations que dans la formule I, des groupes hydroxyles et/ou acyles et/ou alcynyles terminaux présents étant éventuellement protégés, et $K^1$ a la signification de K ou représente à la fois un atome d'hydrogène et un groupe hydroxyle protégé) à l'action d'un agent acide pouvant libérer une ou plusieurs fonctions protégées et éliminer sélectivement le groupe hydroxyle à la position 5α avec formation de la double liaison 4(5), ainsi que, en présence de groupes protecteurs éliminables seulement en milieu basique, avant ou après l'action de l'agent acide, à l'action d'un agent basique, puis on oxyde un groupe hydroxyle éventuel de $K^1$, si l'on veut on estérifie ou éthérifie des hydroxyles libres éventuellement présents à la position 17 et/ou sur $R^3$, puis si on le souhaite également on fait réagir le produit formé de formule I dont X est un atome d'oxygène avec du chlorhydrate d'hydroxylamine en présence d'amines tertiaires.

**4.** Préparations pharmaceutiques caractérisées en ce qu'elles comprennent des composés des revendications 1 et 2.

**5.** Emploi des composés des revendications 1 et 2 pour la fabrication de médicaments.